# EUROPEAN PATENT APPLICATION

(11) **EP 3 832 357 A1**
(43) Date of publication of application: **09.06.2021**
(21) Application number: 20209513.9
(22) Date of filing: 24.11.2020
(51) Int. Cl.: G02B 1/18, B32B 7/12, B32B 27/22, C08J 7/054, C08J 7/056

(54) **ANTI-FOG LENS COVERING SYSTEMS**

(30) Priority: 02.12.2019 US 201916700971
(71) Applicant: Ripclear LLC, Jackson Heights, New York 11372 (US)
(72) Inventor: HINES, Zachary, Jackson Heights, NY New York (US); DOHERTY, Ryan, Jackson Heights, NY New York (US)
(74) Representative: Windsor, Louise

(57) **Abstract**

A lens protection systems that include an optically clear anti-fog film which remains on the lens during use, and in some implementations carrier layers that are used to protect the film during shipment and/or assist an end user with applying the anti-fog film to the lens. These systems are suitable for use with a wide variety of optical lenses, for example eyewear, including but not limited to ski goggles, tactical goggles, diving goggles, anti-fog eyewear such as safety glasses, sun glasses, helmet visors (e.g., for football, hockey, or motorcycle helmets), and paintball masks.

## Description

### BACKGROUND

Lenses of eyewear, for example goggles used in sports and outdoor activities and protective eyewear (e.g., safety glasses), are subject to fogging, due to exertion by the user and/or environmental conditions. Fogging can cause safety issues, such as falls, collisions, loss of eyesight, or even death from injury, and at best results in annoyance and inconvenience. Wiping lenses to remove fogging can result in scratching or damage to the lens, particularly if a lens cleaning cloth is not available to the user. Removal of eyewear due to fogging can increase eye injuries and result in loss of performance or accidents due to poor vision.

For some users, such as workers who must go from a cold environment to a warmer environment frequently, lens fogging can be a serious problem that can result in a loss of productivity as well as hazard and frustration. For users involved in strenuous activities, such as military personnel and athletes, fogging limits performance and can force the user to terminate the activity.

### SUMMARY

The present disclosure features anti-fog lens covering systems, for example, for preventing or reducing fogging of eyewear lenses during use. The systems include an anti-fog film that is adhered directly to a surface of the lens and remains on the lens during use. In some implementations, the systems include one or more carrier layers that may be used to assist an end user or a manufacturer with applying the anti-fog film to the lens. When used with eyewear, the anti-fog film may be applied to an inner surface of the lens to prevent fogging of the inner surface (the most common fogging problem), to the outer surface to protect the surface from scratching and other damage as well as preventing fogging of the outer surface, or to both surfaces.

These systems are suitable for use with a wide variety of eyewear, including but not limited to ski goggles, prescription goggle lenses, tactical goggles, sun glasses, corrective prescription glasses, diving goggles, protective industrial eyewear such as safety glasses, firefighting protective eyewear, welding shields, helmet visors (e.g., for football, hockey, or motorcycle helmets), and paintball masks. While primarily intended for eyewear, the systems disclosed herein can be utilized to prevent fogging of other types of lenses such as the lenses of drone cameras, professional grade cameras or video cameras, such as those commercially available from Canon®, Nikon®, or GoPro®, and camera accessories having optically clear surfaces, such as camera loupes and viewfinders. The systems disclosed herein can also be used to prevent fogging of hunting scopes, gun scopes, telescopes, binoculars and the like. The systems can also be used with optical sensors, in automotive applications such a headlight lenses, and in many other applications where lenses are utilized.

In some implementations the systems are semi-customized, for example, some systems are shaped to fit within the frame of a ski goggle without obscuring the vents of the goggle, or to cover a lens from edge to edge. In other implementations the systems are universally fitted to fit a particular type of lens, for example the anti-fog film can be sized to cover a major portion of the surface of the face shield of a helmet. In some factory-applied systems, the anti-fog film is applied uncut to a sheet stock and then the film and sheet stock are cut together to form the lens.

In some preferred implementations, the anti-fog film that remains on the lens has a very high degree of transparency and light transmission, such that the visual acuity and other optical characteristics obtainable with the eyewear are substantially unchanged by the presence of the layer. Some protective films are configured to allow the lens to which they are applied to pass optical tests such as the ANSI Z87.1-2010 rating or other related tests. In some cases, a lens with the protective film in place will meet the MIL-PRF-32432 Optical Distortion specification, and/or the ANSI Z87.1 haze, prism power, resolving power, refractive power and astigmatism standards.

The anti-fog film also preferably meets or surpasses established quantitative anti-fogging standards, such as European N mark testing standards EN166 Resistance to Fogging (clause 7.3.2) and EN168 and ASTM F659. Both EN166 and ASTM F659 assess fogging quantitatively through measurement of the change in light transmittance through the lens. Both EN166 and ASTM F659 use 80% of the original transmittance as a minimum acceptable level. To achieve the EN166 N mark, the change in transmittance must remain above 80% for a minimum of 8 seconds. In the ASTM standard, a minimum of 30 seconds must be achieved.

In one aspect, the disclosure features an anti-fog lens covering system comprising an optically transparent anti-fog film, comprising a hydrophilic sheet material carrying a layer of an optically transparent adhesive.

Some implementations include one or more of the following features.

The anti-fog film may comprise a superhydrophilic polymer (a polymer having a Water Contact Angle of less than 10 degrees), such as cellulose diacetate or cellulose diacetate modified with plasticizer. In some preferred implementations the anti-fog film comprises cellulose diacetate modified with one or more plasticizers.

The adhesive may be configured to removably adhere the film to a surface of an optical lens without leaving a visible residue on the lens when removed therefrom. Alternatively, the adhesive may be configured to permanently bond the anti-fog film to a surface of an optical lens. The anti-fog film may be shaped to fit within a frame in which an eyewear lens is mounted, or to fit the shape of a non-eyewear lens.

The system may further include at least one outer carrier layer removably adhered to the anti-fog film, e.g., outer carrier layers removably adhered to both sides of the anti-fog film. In some implementations, the outer carrier layers comprise an application layer configured to assist a user in applying the anti-fog film to the lens; and a base layer, configured to provide stiffness to the lens protection system during shipping, retailing, and storage.

Generally, the application layer is adhered to the anti-fog film by a pressure sensitive adhesive carried by the application layer, e.g., a silicone adhesive. The adhesive carried by the anti-fog film may provide a watertight seal against the lens surface, or, alternatively, a permeable seal. The layer of adhesive carried by the anti-fog film may have a thickness of, for example, about 10 to 30 µm.

In some cases, the anti-fog film has a thickness of from about 0.01 to 1.0 mm, for example from about 100 µm to 1000 µm, or from about 100µm to 600µm.

In another aspect, the present disclosure features an anti-fog lens covering system comprising an optically transparent anti-fog film, the film comprising a sheet material carrying an anti-fogging optically transparent hydrophobic coating on one surface and a layer of an optically transparent adhesive on an opposite surface.

Some implementations include one or more of the following features.

The adhesive may be configured to removably adhere the film to a surface of an optical lens without leaving a visible residue on the lens when removed therefrom.

The anti-fog film may comprise a thermoplastic urethane or polyethylene terephthalate. The coating may be superhydrophilic. In some implementations, the coating is silane-based.

In another aspect, the invention features a method of preventing fogging of a lens, the method comprising applying an optically transparent anti-fog film directly to the surface of a lens, the film comprising a sheet material carrying a layer of an optically transparent adhesive.

Some implementations include one or more of the following features.

The lens may be an eyewear lens. The adhesive may be configured to removably adhere to a surface of the eyewear lens without leaving a visible residue on the lens when removed therefrom.

The method may include, prior to applying the anti-fog film, removing a base layer from the anti-fog film, exposing the adhesive carried by the anti-fog film. The method may further include, after applying the anti-fog film, removing an application layer from the anti-fog film, the application layer being adhered to the anti-fog film by a pressure sensitive adhesive carried by the application layer.

In some implementations the method includes, prior to removing the application layer, smoothing the anti-fog film against the lens surface by applying pressure to the application layer.

The method may further include removing the anti-fog film from the lens and applying a new anti-fog film to the lens.

The anti-fog film may comprise a hydrophilic polymer film or, alternatively, a thermoplastic polyurethane or polyethylene terephthalate film having a hydrophobic coating.

The anti-fog film may be applied to the lens by an end user of eyewear containing the lens, or, alternatively, by a manufacturer of the lens or eyewear.

If the anti-fog film is to be applied to the outer surface of a lens, the anti-fog film may carry a scratch resistant coating layer on a surface opposite the surface on which the adhesive is carried, for example to extend the useful life of the anti-fog film if the anti-fog film is formed of a material that is susceptible to scratching and is to be used to protect the lens as well as preventing fogging. Suitable scratch resistant coatings do not unacceptably degrade the anti-fog properties of the anti-fog film.

In some implementations, the system further includes application tabs extending from the carrier layers. The carrier layers may in some cases be formed of polyethylene terephthalate or polyethylene, but may include or be formed of other materials to meet the requirements of a particular application.

In some cases, for example when the anti-fog film is to be applied to the lens by a manufacturer rather than an end user, the system may include only a single carrier layer.

The disclosure also features kits of accessories that may be supplied with or separately from the anti-fog lens covering systems, e.g., lens cleaning kits, that allow the films to be applied in a manner so that the lens with the film will exhibit the quality and functionality required to meet lens test specifications.

### DESCRIPTION OF THE DRAWINGS

FIG. 1 is a perspective view of an anti-fog lens covering system according to one implementation.
FIG. 1A is a highly enlarged cross-sectional view of the anti-fog lens covering system of FIG. 1.
FIG. 1B shows an alternate embodiment in which the application layer is omitted from the system.
FIG. 2 is a perspective view of the anti-fog film of the anti-fog lens covering system shown in FIG. 1, in use on goggles. FIGS. 2A and 2B are side and top views, respectively, of the anti-fog film in use, illustrating the "spherical" shape of the goggle lens.
FIGS. 3A-3E are diagrammatic views showing steps in the application of the anti-fog lens covering system shown in FIG. 1 to a pair of ski goggles.
FIG. 4 is a front view of an anti-fog film having an alternative shape.
FIG. 5 is a front view of an anti-fog film having a different shape, positioned on a football helmet visor.
FIG. 6 is a rear perspective view of a pair of sunglasses having an anti-fog film adhered to the inner surface of the lenses.
FIG. 6A is a partial perspective view of the sunglasses shown in FIG. 6, cut away and enlarged to show the lens and anti-fog film more clearly.
FIG. 7 is a perspective view of half of a pair of sunglasses, showing an anti-fog layer mounted on the inner surface and a protective layer mounted on the outer surface.

### DETAILED DESCRIPTION

### Manually-applied Systems

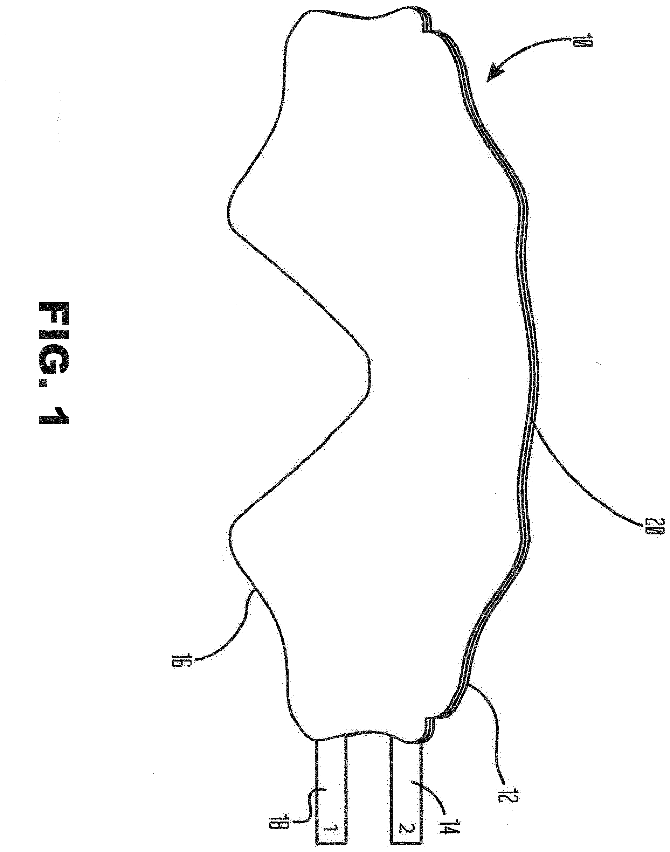

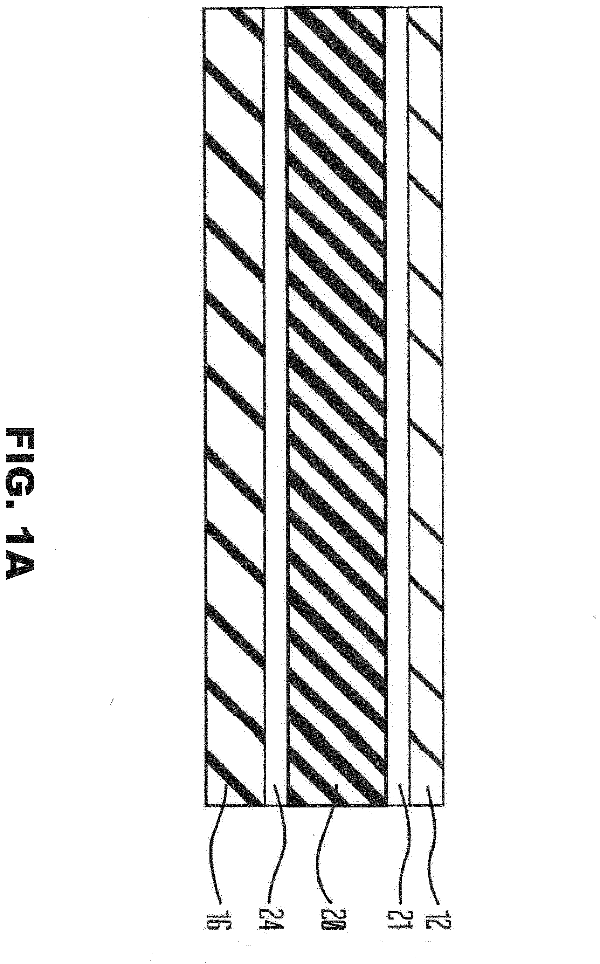

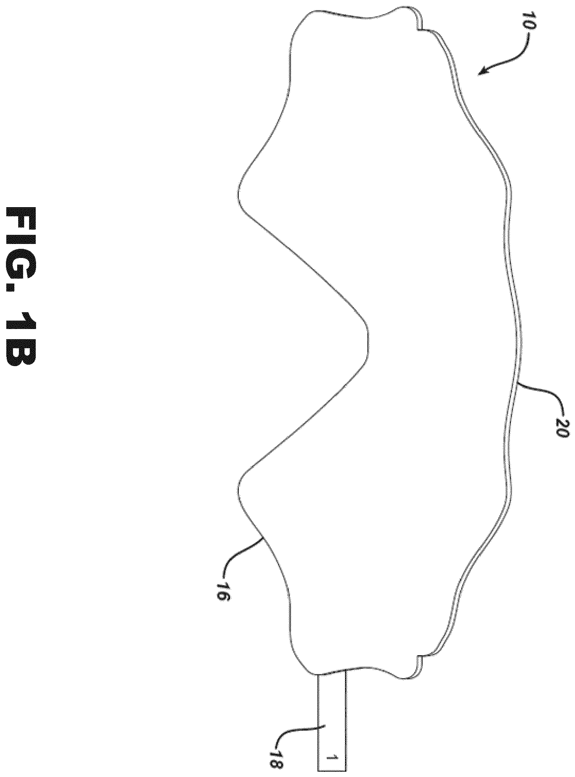

**FIGS. 1-1A** show an implementation in which the anti-fog lens covering system is provided as a laminate that includes carrier layers provided on opposite sides of an anti-fog film that remains on the lens. This multi-layer arrangement facilitates manual application of the anti-fog film to a lens, e.g., by an end user or by an operator performing manual application on a factory assembly line.

Referring to **FIGS. 1** and **1A**, an anti-fog lens covering system **10** according to one implementation includes an application layer **12**, having an application tab **14** extending from one corner, a base layer **16**, having an application tab **18** extending from one corner, and an anti-fog film **20** interposed between the application layer and base layer. As will be discussed in further detail below, the anti-fog film **20** is applied to the lens and prevents fogging of the lens during use, while the application layer **12** assists in applying the anti-fog film to the lens (as well as protecting the non-adhesive surface of the anti-fog film during shipping and storage), and the base layer provides support to the other two layers and prevents creasing or other damage to the layers prior to use while also protecting the adhesive carried by the anti-fog film from contamination. An alternate embodiment is shown in **FIG. 1B**, in which the application layer **12** is omitted.

The anti-fog layer may be applied to the inner surface of the lens, the outer surface of the lens, or both. Applying an anti-fog layer to both the inside and outside surfaces can be useful in applications where the lens is exposed to rapid temperature changes, such as when workers are going in and out of refrigerated environments. If the anti-fog layer is applied to the inner surface only, a protective layer may be applied to the outer surface. Suitable protective layers are described in U.S. Patent Nos. 10,228,494 and 9,442,306, the full disclosures of which are incorporated by reference herein.

An adhesive layer **24** (**FIG. 1A**) is provided on the surface of the anti-fog film **20** that faces the base layer **16**. This adhesive layer **24** initially adheres the base layer to the anti-fog film, and then during application the adhesive stays with the anti-fog film and allows the anti-fog film to be removably adhered to the lens. Because the adhesive layer **24** will be present on the lens, the adhesive used is optically transparent and is formulated to be easily removable from the lens by the user, without leaving an adhesive residue and without damaging the lens. It is also generally preferred that the adhesive provide a secure bond with the lens. In some cases, particularly when the eyewear is to be used in an outdoor environment, it may be desirable for the bond provided by the adhesive to be airtight and watertight. However, in other implementations the adhesive bond can be permeable. Adhesive layer **24** is also preferably formulated to contribute to optical clarity by preventing creasing or visible marks from application of pressure while applying the anti-fog film to the lens.

The application layer is adhered to the anti-fog film by a thin layer **21** of a low adhesion pressure sensitive adhesive provided on the application layer. This adhesive layer is configured to have sufficiently low adhesion to allow the application layer to be very easily stripped from the anti-fog film, without removing the anti-fog film from the lens, while having sufficiently strong adhesion to hold the application layer in place on the anti-fog film during application of the anti-fog film to the lens. The adhesive remains on the application layer when the application layer is removed, leaving the anti-fog film substantially free of adhesive residue, i.e., such that the surface is non-tacky and the transparency of the anti-fog film is not impaired.

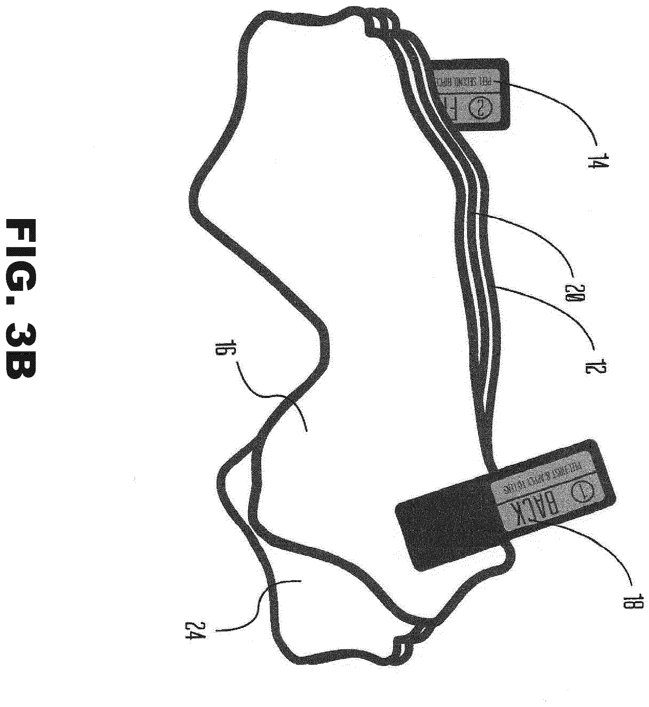

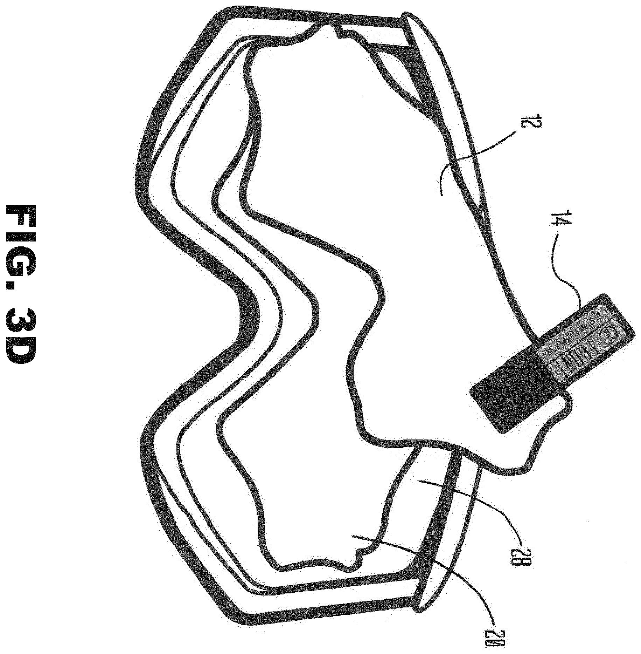

The application layer may include application tabs **14**, **18**, which allow the anti-fog film **20** to be positioned on a lens **28** (**Claim 18**) without the user's fingers contacting the adhesive layer **24** or leaving fingerprints on either surface of the anti-fog film. The application layer **12** allows the anti-fog film to be applied to the lens without bubbles or creases being present between the lens and the anti-fog film, and without dust or other debris being trapped under the anti-fog film. The application tabs provide the user with a portion to grasp, and may include indicia (e.g., "1" and "2", as shown, or instructions, such as shown in **FIGS. 3B** and **3D**) to help the user apply the anti-fog film. For example, the numbering on the tabs in **FIG**. **1** indicates to the user which layer should be removed first. The application tabs also assist the user with repositioning the anti-fog film if the user does not like how it is aligned on the lens, and/or lifting a portion of the film to remove trapped debris or air.

The anti-fog film can be configured to be conformable to the lens shape when applied by an end user, in which case the film may be more or less flexible depending on the complexity of the shape. For example, for end-user application generally a stiffer film may be used if the lens is flat or curved in only a single direction than if the lens is curved in more than one direction (e.g., a spherical lens) or has other characteristics that require a very conformable film.

The anti-fog film can also be configured to provide a desired degree of protection to the lens, which will depend on the operating environment (ski slope, desert conditions, industrial use, etc.) and desired usable life.

Because it is the layer that will remain on the lens, the anti-fog film is transparent, and preferably has optical characteristics that are matched to or compatible with those of the lens.

The application layer generally needs to be sufficiently soft and flexible to conform to the lens during application, while providing enough structural stability to the anti-fog film to prevent wrinkling, creasing, scratching, contamination and other potential damage to the anti-fog film during application.

The base layer is configured to protect the anti-fog layer prior to application, e.g., during shipping and storage. The base layer is generally sufficiently stiff to prevent bending or flexing of the system in more than one direction during shipping and storage.

The application and base layers do not need to be transparent, although it may be desirable for the application layer to have some transparency to allow visualization of the underlying anti-fog film during application. Either or both of the layers may be provided with graphics or other indicia, e.g., advertising or marketing information.

The various layers and suitable materials for the layers will be discussed in further detail in the Materials section below.

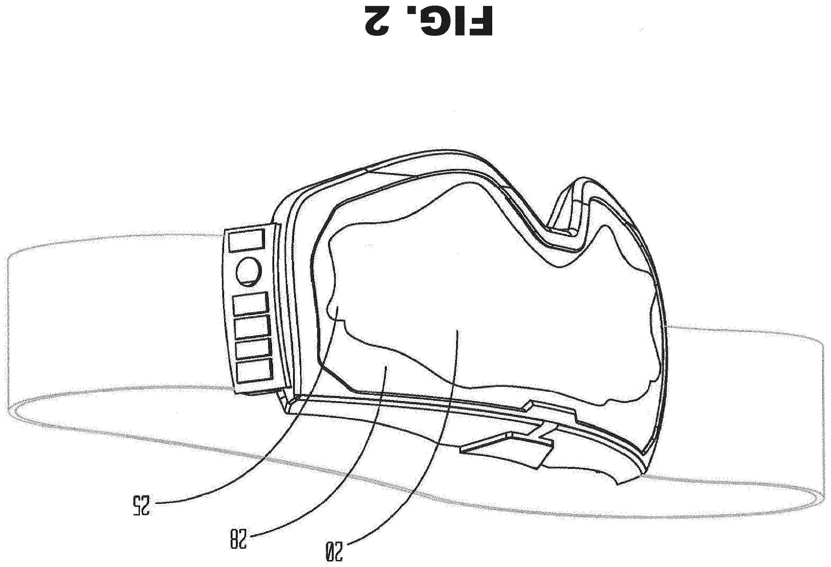

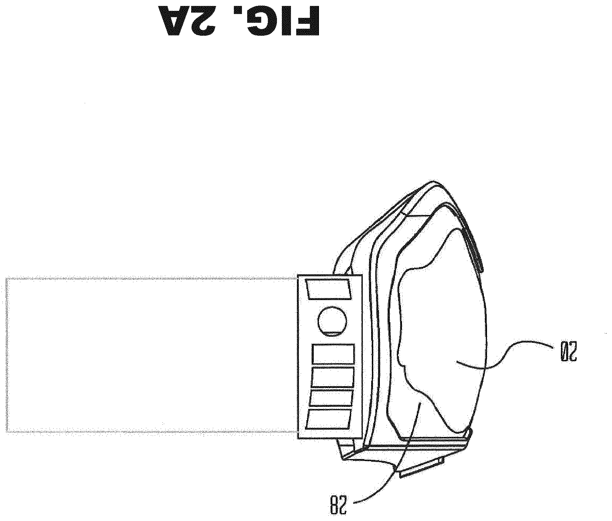

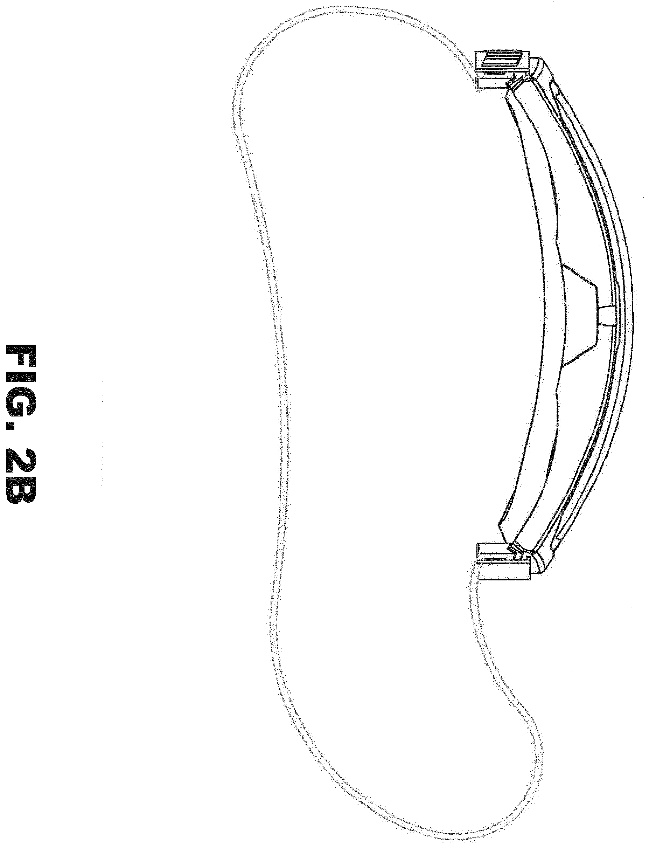

Referring to **FIG. 2**, when the system is to be used on a spherical lens the peripheries of the anti-fog film and the application layer may in some cases be curved. This can help to prevent bunching and creasing of the edge of the anti-fog and application layers as the system is applied to the lens by an end-user. Alternatively, the anti-fog film can be sized to be sufficiently smaller than the lens so that a curved edge is not needed due to the margin between the edge of the film and the frame.

The anti-fog film may include a tab **25** (**FIG. 2**) that allows the user to more easily slip a fingernail under the film to detach it from the lens. For ease of manufacturing it is generally preferred that the base layer have the same outline as the other layers; however, if desired the base layer can have a different shape, so long as it adequately supports the anti-fog film and is removable therefrom by the user.

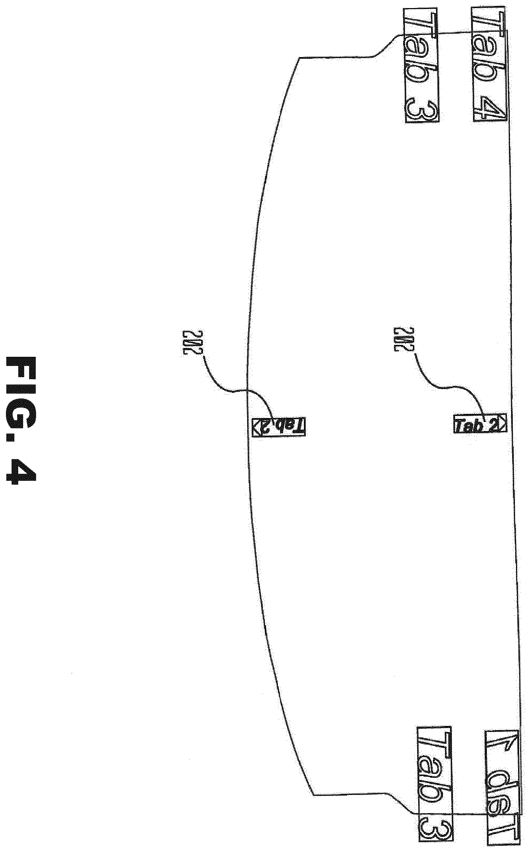

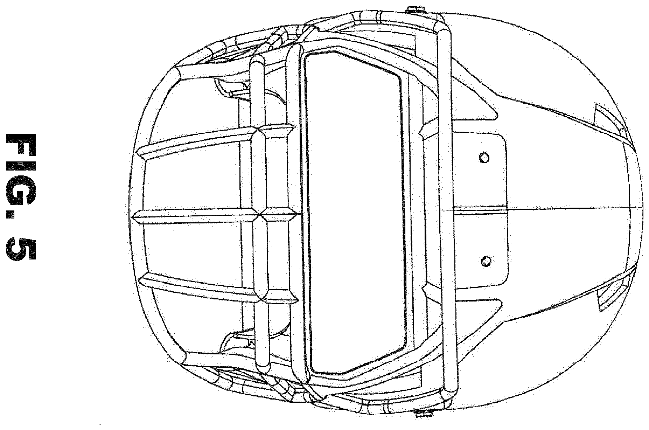

Examples of anti-fog films applied to non-spherical lenses are shown in **FIGS. 4** and **5**. When the anti-fog film is to be manually applied it is preferred that the anti-fog film be sized so that it will fit within the frame of the eyewear and not obscure vents or cover the frame or other non-lens portions of the eyewear. Depending on the material used for the anti-fog film, it may be necessary to supply the system to the user in a pre-formed condition (similar to a contact lens) to conform the anti-fog film to the surface of a spherical lens. Alternatively, the user can be instructed to lightly heat the system prior to application (e.g., with a hairdryer or heat gun) if the material of the anti-fog film requires heating in order to be sufficiently formable (for example, if the anti-fog film is cellulose diacetate.)

### Factory Application

The anti-fog film can be applied in a factory setting, either by a manufacturer of the end product (e.g., eyewear, camera, etc.), a manufacturer or a lens, or a manufacturer of a stock material that will be used to form a lens. When the film is applied in a factory using application equipment, one or both of the carrier layers discussed above can be omitted.

If the anti-fog film is to be applied by a manufacturer, the material of the anti-fog film can be selected to allow the film to be thermoformed onto the lens or laminated or thermoformed onto an optical stock that is used to form the lens. Applying the anti-fog film to planar optical stock allows the anti-fog film to be readily used on lenses of any shape and size, since the anti-fog film will be thermo-formed with the lens as a supporting substrate.

Various techniques can be used to apply the film to the lens material. For example, the film can be laminated onto a planar lens stock material, e.g., by supplying the film on a roll and laminating the film to the stock using nip rolls as is well known in the art. The film can be applied to non-planar lens blanks (e.g., toric, spherical or cylindrical) using other methods such as vacuum suction, thermoforming or manual application.

The sheet stock or lens blanks can be cut to the desired shape using any desired technique, for example using lens edger such as those commercially available from MEI Systems and Santinelli.

### Materials

The anti-fog film is formed of a
material that is highly transparent, and in some cases optically transparent.

Preferred materials include superhydrophilic polymers (polymers having a Water Contact Angle (WCA) of less than 10 degrees), such as cellulose diacetate and cellulose diacetate modified with plasticizers. Advantageously, cellulose diacetate films are biodegradable and compostable. In some cases, the WCA is less than 2 degrees or is zero.

Preferably, the anti-fog film has mechanical properties that allow it to be applied to the lens by a desired method (e.g., by an end user, or by a lens or product manufacturer) without tearing or damaging the film and that provide the film with sufficient durability during use. Some anti-fog films have a tensile strength (ASTM D882) of from about 60 to 90 Nmm⁻², an elongation at break (ASTM D882) of about 30 to 60%, and an E-Modulus (ASTM D882) of about 1600-1900 Nmm⁻².

Some anti-fog films have a softening temperature of at least 130°C, e.g., 140°C, allowing the anti-fog film to be thermoformed with a lens material. In some implementations the softening temperature may be considerably lower, e.g., at least 35°C.

The anti-fog film may in some cases have a thickness of less than 1 mm, e.g., less than 0.5 mm, or less than 0.2 mm, e.g., about 0.15 mm. In some implementations the thickness of the anti-fog layer will be from about 0.01 to 1 mm, for example from about 100 µm to 1000 µm, or from about 100µm to 600µm. The thickness of the layer will depend upon the material used, the required durability and useful life of the layer, and the expected use conditions.

The application layer will generally be formed of a flexible thermoplastic sheet material, for example a polyethylene (PE) or polyethylene terephthalate (PET) polymer. Other flexible thermoplastics may also be used. It is generally preferred that the material be one to which pressure sensitive adhesives will adhere. In some implementations, e.g., when the system is to be used on a spherical lens, the material has a low durometer, e.g., less than 60 Shore D or even less than 40 Shore D, for example from about 10 to 60 Shore D. In this case, the PET may be formulated with additives, e.g., plasticizer, to reduce its hardness. This low durometer rating provides the application layer with sufficient flexibility to allow it to conform to a curved, toric, or spherical (double-curved) lens during application of the anti-fog film. The application layer is generally transparent or translucent to allow the lens surface to be seen during application but could be opaque if desired. The thickness of the application layer will depend on whether the system is being applied to a spherical goggle -- in which case the application layer needs to be sufficiently thin to conform to the shape of the lens along with the anti-fog film -- or another shape of lens, in which case the application layer can be thicker if desired. In some cases, the application layer has a thickness in the range of about 0.01 to 1 mm, e.g., from about 0.05 to 0.3 mm. If the lens is relatively flat or gently curved, the application layer can be thicker, with generally the only constraints being cost and optical clarity.

The application tab may be formed of the same material as the application layer, for example if it is desired that the application tab be integral with the application layer, or may be formed of a different material and bonded, e.g., adhered or welded, to the application layer. In the latter case, the application tab may be formed of a stiffer material to give the user a better grasp on the application layer, and/or may be formed of a material that is easy to print so that the indicia/instructions can be easily applied to the tab. If the application tab is formed integrally with the application layer the indicia/instructions may be applied by adhering a label to the tab.

The base layer may be of any sheet material having sufficient stiffness to protect the other layers during shipping and storage. For example, the base layer may be formed of PET or another thermoplastic having a relatively high durometer, e.g., at least 30 Shore D, for example from about 30 to 90 Shore D. The base layer may also be formed of materials other than plastics, e.g., release papers. The base layer may have a thickness similar to that of the application layer or may be thicker if desired. Generally, the only limitation on the thickness of the base layer is cost.

The adhesive that is carried by the anti-fog film is pressure sensitive and is preferably optically transparent, e.g., with at least 99% optical clarity. Suitable adhesives include, for example, pressure sensitive silicone adhesives such as those commercially available from Dow Corning, e.g., Dow Corning 7651 high performance optical grade silicone pressure sensitive adhesive. In some implementations the adhesive may be a solvent-based acrylic silicone adhesive. In addition to providing a good seal between the lens and anti-fog film, preferred adhesives retain their transparency when pressure is applied to adhere the layer to the lens, are repositionable, will not damage the lens or remain on the lens when the anti-fog film is removed, and adhere to the material used for the anti-fog film. The peel strength of the adhesive is generally selected so that the anti-fog film will remain on the lens during the intended use period but can be removed by the user inserting a fingernail under the edge of the film and peeling the film back. Alternatively, the adhesive may in some cases be formulated to permanently, rather than removably, adhere the film to the lens. In some cases, the adhesive layer **24** has a thickness of about 0.02 to 0.03 mm.

In some cases, the adhesive carried by the anti-fog film is selected to allow the Ballistic Fragmentation performance of the lens to remain substantially unaltered after the lens has been exposed to the adhesive for a period of time, e.g., at least 1 day, at least 1 week, or at least 1 month, or at least 1 year from initial application.

The adhesive used between the base layer and anti-fog film may in some cases be formulated to have a relatively lower peel strength, and/or be provided in a thinner layer, so that the base layer can be easily separated from the anti-fog film.

### Applying the Anti-foa Film (Manual Application)

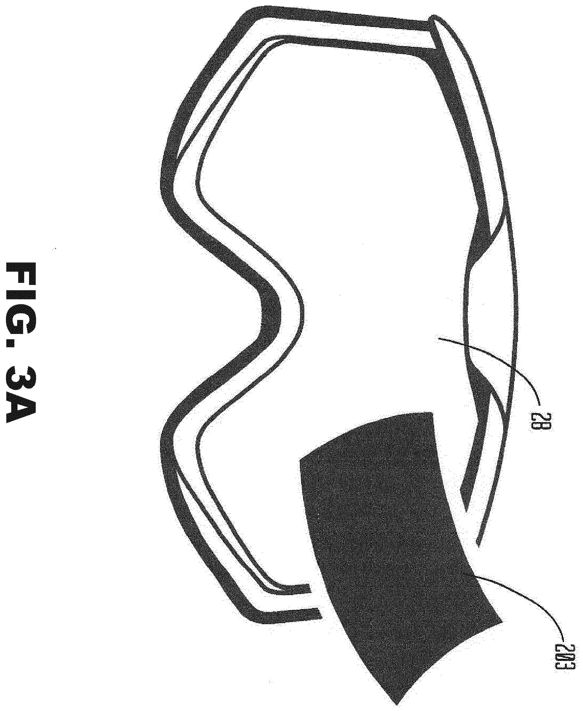

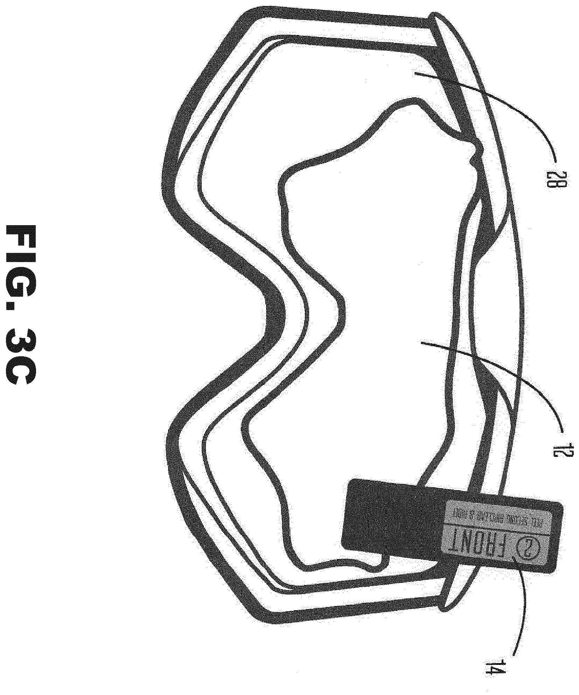

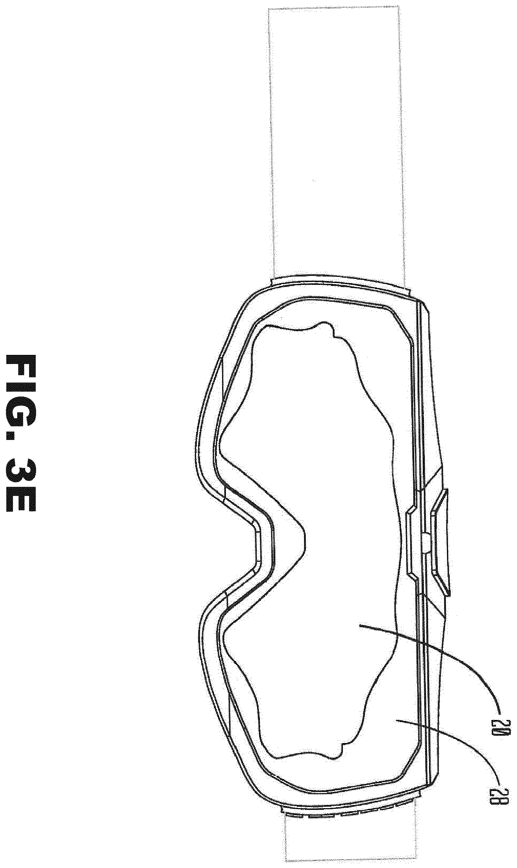

The anti-fog film is applied to the lens as shown in **FIGS. 3A-3E**. First, it is important to clean the lens (**FIG. 3A**). Once the lens is clean and substantially free of dust and debris, the user peels off the base layer (**FIG. 3B**), holding the system by the two tabs **14, 18**. This exposes the adhesive layer **24** on anti-fog film **20**. The user is then left holding the application layer **12** and the attached anti-fog film **20** by the application tab **14**. (Or, in the case of a large anti-fog film, the more substantial tabs **3**, shown in **FIG. 4**). The user lines the layers up with the goggle in the desired orientation, and then gently touches the adhesive layer **24** of the anti-fog film **20** to the goggle surface (**FIG. 3C**). Next, starting from the middle, the user uses a soft cloth to press the application layer towards the lens surface, adhering the anti-fog film to the surface while pushing any trapped air outwards from the center. If necessary, the application layer and attached anti-fog film can be peeled up during application to remove any trapped air or dust particles. Last, the user grasps the application tab of the application layer and peels the application layer from the anti-fog film (**FIG. 3D**), leaving the anti-fog film firmly attached to the lens surface by the adhesive **24** (**FIG. 3E**). The outer surface of the anti-fog film is smooth and non-tacky, due to the attachment of the application layer to the anti-fog film by pressure sensitive adhesive **21**, which stays with the application layer when it is removed.

In some implementations, the system includes tools and instructions for cleaning the lens so it is dust and dirt free and to assist the user in applying the anti-fog film to the lens. For example, the system may include application instructions, an alignment sticker (e.g., centering stickers **202** provided on the application layer, as shown in **FIG. 4**), a wet cleaning wipe or lens cleaning spray for cleaning the lens (e.g., wipe **203** in **FIG. 3A**), a microfiber cloth for pressing the application layer towards the goggle surface, and a dust removal sheet (for dust removal before application) and/or a dust removal sticker to help the user remove any dust or debris particles that are trapped during application (by peeling back the layers and dabbing the surface with the sheet before reapplying the layers). These components may be sold or otherwise supplied separate from the anti-fog lens covering system, e.g., the dust removal sheet, dust removal sticker(s), and in some cases the microfiber cloth can be supplied as a separate cleaning kit.

Once it is applied, the anti-fog film will stay in place until removed by the user. In some implementations, the adhesive provides a watertight and airtight seal between the lens and the anti-fog film that will hold the anti-fog film securely in place under whatever environmental conditions are encountered. When the user considers the anti-fog film to be in need of replacement (e.g., due to scratching or loss of anti-fog performance), the user gently inserts a fingernail under an edge of the anti-fog film (e.g., under tab **25** in **FIG. 2**), peels back the layer, and strips it from the lens. The adhesive remains with the anti-fog film, leaving the lens free of adhesive residue. The user can then replace the anti-fog film by repeating the steps described above.

The time that the anti-fog film may be used on a lens prior to removal and replacement will vary depending on a variety of factors, including the requirements of the user and the usage environment. For some applications the anti-fog film may be used for three months or more (in some cases as much as multiple years) of daily use of the eyewear, while in other scenarios it may be desirable to replace the anti-fog film after a few uses or even a single use of the eyewear. Advantageously, the system is easy to apply and relatively inexpensive, and thus a new anti-fog film can be applied as often as desired.

### Other Embodiments

A number of embodiments have been described. Nevertheless, it will be understood that various modifications may be made without departing from the spirit and scope of the disclosure.

While superhydrophilic materials, such as modified cellulose diacetate films, are generally preferred for use as the anti-fog film, alternatively the anti-fog film may be formed of one of the materials used for the protective layer in U.S. Patent Nos. 10,228,494 and 9,442,306, with an optically transparent hydrophobic coating applied to the exposed surface of the material. Hydrophobic coatings that are optically transparent and that will adhere to flexible polymeric films (e.g., thermoplastic polyurethanes and polyethylene terephthalate) are well known in the art. In some preferred implementations the coating is a superhydrophobic coating, i.e., the coating has a Water Contact Angle (WCA) of greater than 150 degrees. Suitable coatings include, for example, those based on any of the following: manganese oxide polystyrene (MnO₂/PS) nano-composites, zinc oxide polystyrene (ZnO/PS) nano-composites, precipitated calcium carbonate, carbon nano-tube structures, silica nano-coatings, fluorinated silanes, and fluoropolymer coatings. Some of these materials, e.g., silica-based coatings, can be gel-based and can be applied by dipping or spraying the lens material.

If scratch resistant coatings are used in conjunction with hydrophobic coatings, the scratch resistant coating is selected to not inhibit the anti-fog performance of the hydrophobic coating.

As another example, while systems having carrier layers on opposite sides of the anti-fog film are shown in the figures, in some implementations the system can include only the anti-fog film itself, or the anti-fog film with only a single layer to facilitate factory application of the anti-fog film to a lens material.

In some implementations, the adhesive used to adhere the anti-fog film to the lens may be replaced by a different (non-silicone based) pressure-sensitive adhesive and/or by electrostatic charge.

While goggles have been discussed above, the systems may be used on many other types of eyewear. For example, the system may be shaped to fit on the eyeshield of a helmet, such as the football helmet shown in **FIG. 5**, or other types of eyewear lenses such as those of sunglasses or safety glasses. The systems may be used on lenses of all shapes, including cylindrical, spherical, and toric lenses.

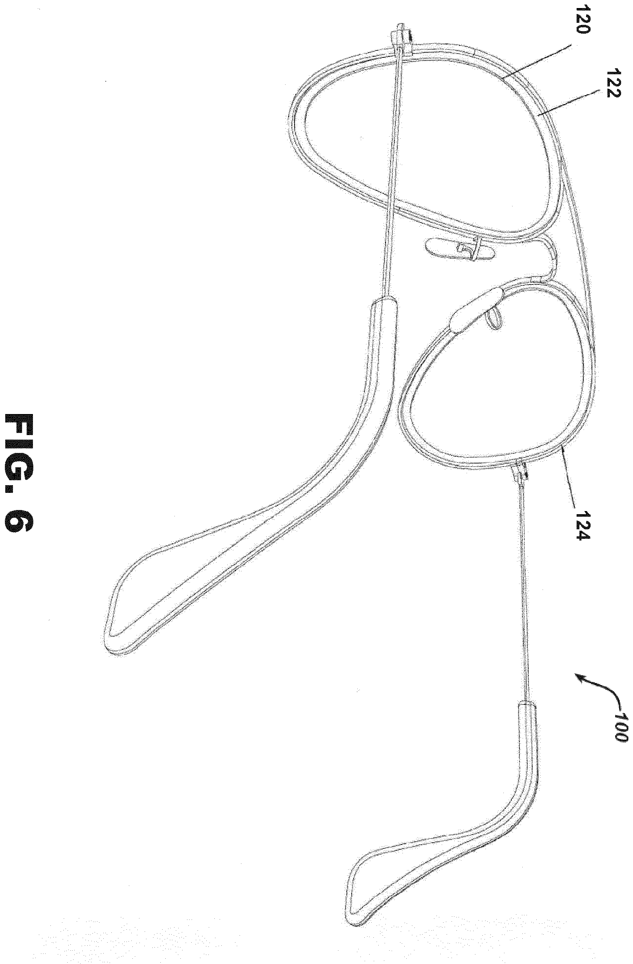

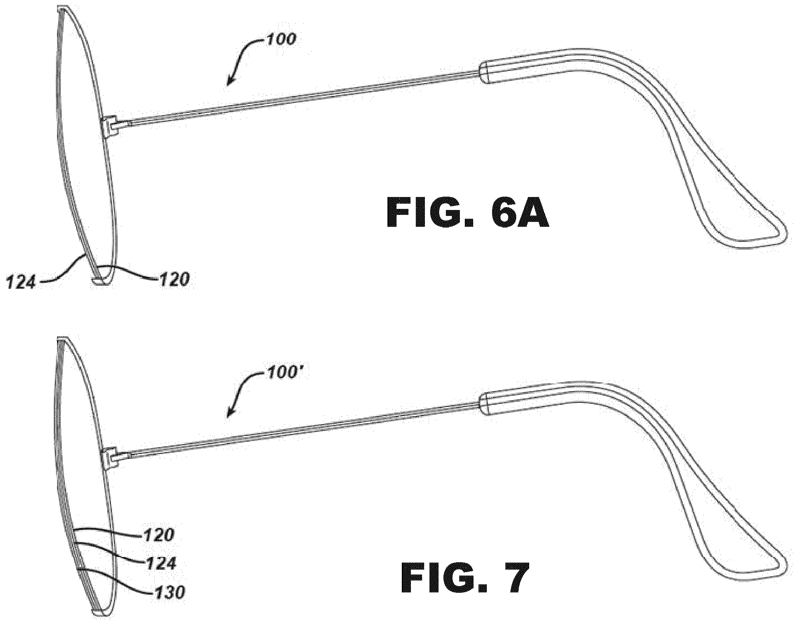

Examples of anti-fog films used on sunglasses are shown in **FIGS. 6-7**. In **FIGS. 6-6A**, a pair of sunglasses **100** includes an anti-fog film **120** adhered to the inner surface **122** of the lenses **124**.

**FIG. 7** is a perspective view of half of a pair of sunglasses **100**, showing an anti-fog layer **120** mounted on the inner surface of lens **124** and a protective layer **130** mounted on the outer surface of lens **124**.

Accordingly, other embodiments are within the scope of the following claims.

The invention includes the following numbered embodiments: -
1. An anti-fog lens covering system comprising:
   an optically transparent anti-fog film, comprising a hydrophilic sheet material carrying a layer of an optically transparent adhesive.
2. The anti-fog lens covering system of embodiment 1, wherein the adhesive is configured to removably adhere the film to a surface of an optical lens without leaving a visible residue on the lens when removed therefrom.
3. The anti-fog lens covering system of embodiment 1 or embodiment 2, wherein the hydrophilic sheet material is superhydrophilic.
4. The anti-fog lens covering system of any preceding embodiment, wherein the anti-fog film comprises cellulose diacetate.
5. The anti-fog lens covering system of any preceding embodiment, wherein the anti-fog film is shaped to fit within a frame in which an eyewear lens is mounted.
6. The anti-fog lens covering system of any preceding embodiment, further comprising at least one outer carrier layer removably adhered to the anti-fog film.
7. The anti-fog lens covering system of embodiment 6, further comprising outer carrier layers removably adhered to both sides of the anti-fog film.
8. The anti-fog lens covering system of embodiment 6 or embodiment 7, wherein the outer carrier layers comprise an application layer configured to assist a user in applying the anti-fog film to the lens; and a base layer, configured to provide stiffness to the lens protection system during shipping, retailing, and storage.
9. The anti-fog lens covering system of embodiment 8, wherein the application layer is adhered to the anti-fog film by a pressure sensitive adhesive carried by the application layer.
10. The anti-fog lens covering system of any preceding embodiment, wherein the adhesive carried by the anti-fog film comprises a pressure sensitive optical grade silicone adhesive.
11. The anti-fog lens covering system of any preceding embodiment, wherein the adhesive carried by the anti-fog film provides a watertight seal against the lens surface.
12. The anti-fog lens covering system of any preceding embodiment wherein the layer of adhesive carried by the anti-fog film has a thickness of from about 10 to 30 µm.
13. The anti-fog lens covering system of any preceding embodiment wherein the anti-fog film has a thickness of from about 0.01 to 1.0 mm.
14. An anti-fog lens covering system comprising:
   an optically transparent anti-fog film comprising a sheet material carrying an anti-fogging optically clear hydrophobic coating on one surface and a layer of an optically transparent adhesive on an opposite surface.
15. The anti-fog lens covering system of embodiment 14, wherein the adhesive is configured to removably adhere the film to a surface of an optical lens without leaving a visible residue on the lens when removed therefrom.
16. The anti-fog lens covering system of embodiment 14 or embodiment 15 wherein the anti-fog film comprises a thermoplastic urethane.
17. The anti-fog lens covering system of any of embodiments 14 to 16, wherein the anti-fog film comprises polyethylene terephthalate.
18. A method of preventing fogging of a lens, the method comprising:
   applying an optically transparent anti-fog film directly to a surface of a lens, the film comprising a sheet material carrying a layer of an optically transparent adhesive.
19. The method of embodiment 18, wherein the lens is an eyewear lens.
20. The method of embodiment 18 or embodiment 19, wherein the adhesive is configured to removably adhere to a surface of the lens without leaving a visible residue on the lens when removed therefrom.
21. The method of embodiments 18 to 20, further comprising, prior to applying the anti-fog film, removing a base layer from the anti-fog film, exposing the adhesive carried by the anti-fog film.
22. The method of any of embodiments 18 to 21, further comprising, after applying the anti-fog film, removing an application layer from the anti-fog film, the application layer being adhered to the anti-fog film by a pressure sensitive adhesive carried by the application layer.
23. The method of any of embodiments 18 to 22, further comprising removing the anti-fog film from the lens and applying a new anti-fog film to the lens.
24. The method of any of embodiments 18 to 23, wherein the anti-fog film comprises a hydrophilic polymer film or a thermoplastic polyurethane having a hydrophobic coating.
25. The method of any of embodiments 18 to 24, wherein the anti-fog film is applied to the lens by an end user of eyewear containing the lens.

## Claims

1. An anti-fog lens covering system comprising:
an optically transparent anti-fog film, comprising a hydrophilic sheet material carrying a layer of an optically transparent adhesive.

2. The anti-fog lens covering system of claim 1, wherein the adhesive is configured to removably adhere the film to a surface of an optical lens without leaving a visible residue on the lens when removed therefrom.

3. The anti-fog lens covering system of claim 1, wherein
i) the hydrophilic sheet material is superhydrophilic; or
ii) the anti-fog film comprises cellulose diacetate; or
iii) the anti-fog film is shaped to fit within a frame in which an eyewear lens is mounted.

4. The anti-fog lens covering system of claim 1, further comprising at least one outer carrier layer removably adhered to the anti-fog film.

5. The anti-fog lens covering system of claim 1, further comprising outer carrier layers removably adhered to both sides of the anti-fog film.

6. The anti-fog lens covering system of claim 5, wherein the outer carrier layers comprise an application layer configured to assist a user in applying the anti-fog film to the lens; and a base layer, configured to provide stiffness to the lens protection system during shipping, retailing, and storage;
optionally, wherein the application layer is adhered to the anti-fog film by a pressure sensitive adhesive carried by the application layer.

7. The anti-fog lens covering system of claim 1 wherein
i)the adhesive carried by the anti-fog film comprises a pressure sensitive optical grade silicone adhesive; or
ii)the adhesive carried by the anti-fog film provides a watertight seal against the lens surface; or
iii) the layer of adhesive carried by the anti-fog film has a thickness of from about 10 to 30 µm.

8. The anti-fog lens covering system of claim 1 wherein the anti-fog film has a thickness of from about 0.01 to 1.0 mm.

9. An anti-fog lens covering system comprising:
an optically transparent anti-fog film comprising a sheet material carrying an anti-fogging optically clear hydrophobic coating on one surface and a layer of an optically transparent adhesive on an opposite surface.

10. The anti-fog lens covering system of claim 9, wherein the adhesive is configured to removably adhere the film to a surface of an optical lens without leaving a visible residue on the lens when removed therefrom.

11. The anti-fog lens covering system of claim 9 wherein i) the anti-fog film comprises a thermoplastic urethane; or ii) the anti-fog film comprises polyethylene terephthalate.

12. A method of preventing fogging of a lens, the method comprising:
applying an optically transparent anti-fog film directly to a surface of a lens, the film comprising a sheet material carrying a layer of an optically transparent adhesive.

13. The method of claim 12 wherein i) the lens is an eyewear lens; or ii) the adhesive is configured to removably adhere to a surface of the lens without leaving a visible residue on the lens when removed therefrom.

14. The method of claim 12, further comprising, prior to applying the anti-fog film, removing a base layer from the anti-fog film, exposing the adhesive carried by the anti-fog film; optionally, further comprising, after applying the anti-fog film, removing an application layer from the anti-fog film, the application layer being adhered to the anti-fog film by a pressure sensitive adhesive carried by the application layer.

15. The method of claim 12, further comprising removing the anti-fog film from the lens and applying a new anti-fog film to the lens; or wherein
i) the anti-fog film comprises a hydrophilic polymer film or a thermoplastic polyurethane having a hydrophobic coating; or
ii) the anti-fog film is applied to the lens by an end user of eyewear containing the lens.
